(19) Europäisches Patentamt
European Patent Office
Office européen des brevets

(11) EP 3 590 515 A1

(12) EUROPEAN PATENT APPLICATION
published in accordance with Art. 153(4) EPC

(43) Date of publication:
08.01.2020 Bulletin 2020/02

(21) Application number: 18761911.9

(22) Date of filing: 28.02.2018

(51) Int Cl.:
*A61K 31/472* (2006.01) *A61J 1/05* (2006.01)
*A61K 9/08* (2006.01) *A61K 31/4725* (2006.01)
*A61K 47/04* (2006.01) *A61K 47/10* (2017.01)
*A61P 27/02* (2006.01) *A61P 27/06* (2006.01)
*B65D 1/00* (2006.01) *B65D 65/20* (2006.01)

(86) International application number:
PCT/JP2018/007583

(87) International publication number:
WO 2018/159702 (07.09.2018 Gazette 2018/36)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(30) Priority: 28.02.2017 JP 2017037417

(71) Applicant: Kowa Company, Ltd.
Nagoya-shi, Aichi 460-8625 (JP)

(72) Inventor: YAMAKITA, Yoshihiro
Fuji-shi
Shizuoka 417-8650 (JP)

(74) Representative: Schiener, Jens
Wächtershäuser & Hartz
Patentanwaltspartnerschaft mbB
Weinstraße 8
80333 München (DE)

# (54) MEDICINE

(57) To establish a technique for stably formulating an isoquinolin-6-amino derivative as an ophthalmic agent or the like. A pharmaceutical preparation is provided including an aqueous composition containing a compound represented by the following formula (1)

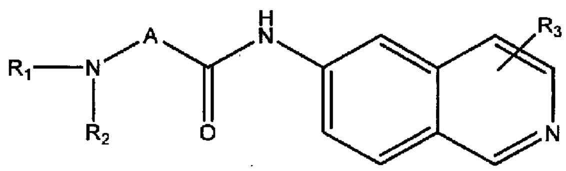

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and the formula (1) also includes a tautomer thereof.) or a salt thereof or a solvate thereof, the aqueous composition being housed in a container formed of a polyester resin or a polyolefin resin.

EP 3 590 515 A1

## Description

Technical Field

The present invention relates to a pharmaceutical preparation and the like.

Background Art

Heretofore, several isoquinolin-6-amino derivatives have been known to be useful in prevention and treatment of eye diseases such as ocular hypertension and glaucoma. Specifically, for example, a compound represented by the following structural formula:

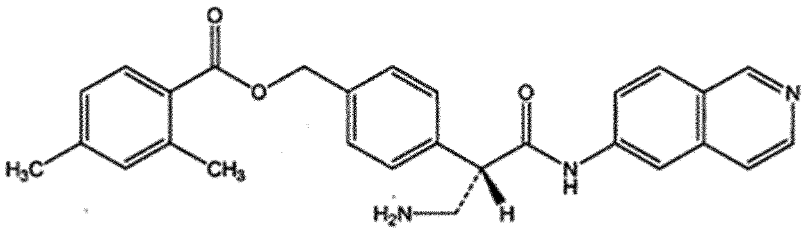

(Chemical Name: [4-[(2S)-3-amino-1-(isoquinolin-6-ylamino)-1-oxopropan-2-yl]phenyl]methyl 2,4-dimethylbenzoate), International Nonproprietary Name: netarsudil, hereinafter sometimes referred to as "netarsudil"), also known as AR-13324, has been reported to have pharmacological effects such as Rho kinase inhibitory effect and norepinephrine transporter inhibitory effect, and be useful in prevention and treatment of eye diseases such as ocular hypertension and glaucoma (for example, Patent Literature 1, and Non Patent Literatures 1 and 2).

Similarly, the compound represented by the following structural formula:

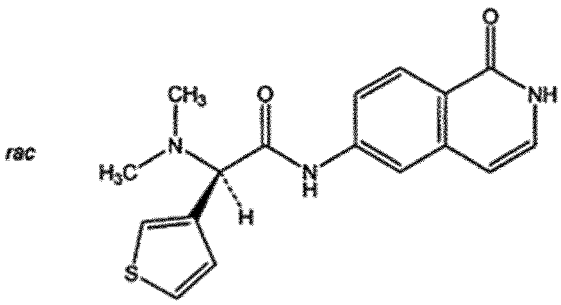

(Chemical Name: rac-(2R)-2-(dimethylamino)-N-(1-oxo-1,2-dihydroisoquinolin-6-yl)-2-(thiophen-3-yl)acetamide), International Nonproprietary Name: verosudil, hereinafter sometimes referred to as "verosudil"), also known as AR-12286, has been reported to have Rho kinase inhibitory effect and be useful in prevention and treatment of eye diseases such as ocular hypertension (for example, Patent Literature 2, and Non Patent Literatures 1 and 3).

Accordingly, it is very useful to establish a technique for stably formulating these isoquinolin-6-amino derivatives, for example, as an ophthalmic agent or the like.

Meanwhile, Patent Literature 3 states that a composition containing netarsudil or verosudil was prepared in a 150 mL plastic container. However, in the literature, each composition was prepared to use in pharmacological studies. The literature does not describe the stability of netarsudil and verosudil at all, neither describes the material and characteristics of the container at all.

Citation List

Patent Literature

Patent Literature 1: JP-A-2012-525386
Patent Literature 2: WO2009/091898

Patent Literature 3: JP-A-2016-515520

Non Patent Literature

Non Patent Literature 1: Bacharach J. et al., Ophthalmology 122(2) 302-7 (2015)
Non Patent Literature 2: Sturdivant JM. et al., Bioorg Med Chem Lett. 26(10) 2475-80 (2016)
Non Patent Literature 3: Williams RD. et al., Am. J. Ophthalmol. 152(5) 834-41 (2011)

Summary of Invention

Technical Problem

An object of the present invention is to establish a technique for stably formulating an isoquinolin-6-amino derivative such as netarsudil or verosudil as an ophthalmic agent or the like.

Solution to Problem

An ophthalmic agent or the like is usually a composition containing water (an aqueous composition). Thus, the present inventor prepared an aqueous composition comprising an isoquinolin-6-amino derivative such as netarsudil and housed the prepared composition in a container to check its storage property. However, unexpectedly, it was revealed that the content of the isoquinolin-6-amino derivative is reduced due to storage at high temperature for a long period of time.

Therefore, the present inventor conducted further extensive studies to improve heat stability of the isoquinolin-6-amino derivative in an aqueous composition. Consequently, the inventor has found that when the aqueous composition comprising the isoquinolin-6-amino derivative is housed in a container formed of a polyester resin or a polyolefin resin, the content reduction at the time of storage at a high temperature is suppressed and a pharmaceutical preparation having improved heat stability can be obtained, and has completed the present invention.

The present invention provides a pharmaceutical preparation comprising: an aqueous composition comprising a compound represented by the following formula (1)

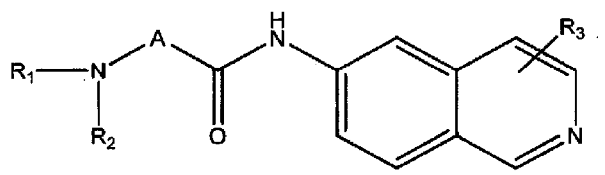

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof, the aqueous composition being housed in a container formed of a polyester resin or a polyolefin resin.

Advantageous Effects of Invention

According to the present invention, it is possible to improve the stability of isoquinolin-6-amino derivatives exemplified by netarsudil in an aqueous composition.

Description of Embodiments

In the present specification, "a compound represented by the formula (1):

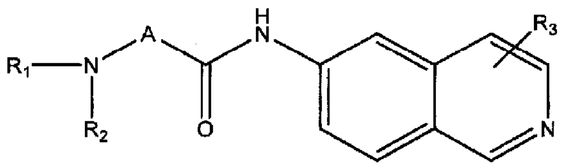

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and the formula (1) also includes a tautomer thereof) or a salt thereof or a solvate thereof" includes not only a compound represented by the formula (1) itself but also a salt or solvate thereof.

Examples of the salt of the compound represented by the formula (1) are not particularly limited as long as those are a pharmaceutically acceptable salts. Specific examples of the salt include an inorganic acid salt such as a hydrochloride, a sulfate, a nitrate, a hydrofluoride, or a hydrobromide; an organic acid salt such as an acetate, a tartrate, a lactate, a citrate, a fumarate, a maleate, a succinate, a methanesulfonate, an ethanesulfonate, a benzenesulfonate, a toluenesulfonate, a naphthalenesulfonate, or a camphorsulfonate, and the hydrochloride and the methanesulfonate are preferred.

Examples of the solvate of the compound represented by the formula (1) or a salt thereof include a hydrate and an alcohol solvate.

Furthermore, when an asymmetric carbon exists in a chemical structure of the compound represented by the formula (1), various stereoisomers can exist, but the configuration of the compound represented by the formula (1) is not particularly limited, and the compound may be a single stereoisomer or a mixture of various stereoisomers in any proportions.

In the specification, when an asymmetric carbon exists in a chemical structure of the compound represented by various formulae, the compound represented by the formula encompasses all of a variety of single stereoisomers and mixtures of the stereoisomers in any proportions, unless otherwise specifies the configuration. Therefore, compounds represented by the formula for which the configuration is not especially specified may be a single stereoisomer or a mixture of various stereoisomers in any proportions.

The compound represented by the formula (1) includes not only a compound directly represented by the formula (1), but also a tautomer of the compound. Specifically, for example, when the formula (1) is the following formula (1a) in which $R_3$ is a hydroxy group, a tautomer thereof (formula (1b)) can exist, then the "compound represented by the formula (1)" includes not only a compound represented by the following formula (1a), but also a compound represented by the formula (1b).

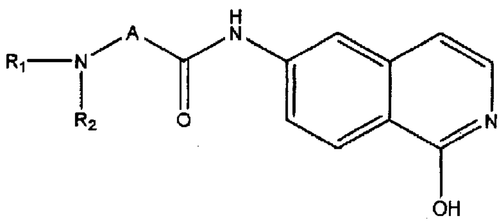

(1 a)

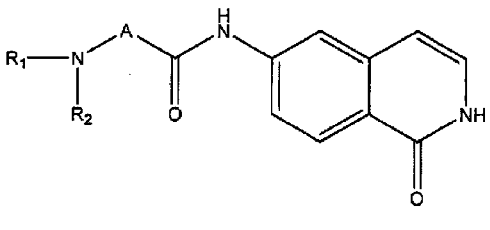

(1 b)

In the present specification, the "$C_1$-$C_4$ alkyl group" means a linear, branched or cyclic alkyl group having 1 to 4 carbon atoms. Specific examples of the "$C_1$-$C_4$ alkyl group" include a methyl group, an ethyl group, a n-propyl group, an isopropyl group, a cyclopropyl group, a n-butyl group, a t-butyl group, and an isobutyl group, and the methyl group is preferable.

In the present specification, the "$C_6$-$C_{10}$ aryl group" means an aryl group having 6 to 10 carbon atoms. Specific examples of the "$C_6$-$C_{10}$ aryl group" include a phenyl group and a naphthyl group, and the phenyl group is preferable.

In the present specification, the "5 to 10-membered heteroaryl group" means a 5 to 10-membered monocyclic, polycyclic or condensed cyclic aromatic heterocyclic group having 1 to 4 heteroatoms selected from the group consisting of an oxygen atom, a sulfur atom, and a nitrogen atom as a ring-constituting atom(s). Specific examples of the "5 to 10-membered heteroaryl group" include a furyl group, a thienyl group, a pyrrolyl group, an oxazolyl group, an isoxazolyl group, a thiazolyl group, an isothiazolyl group, an imidazolyl group, a pyrazolyl group, an oxadiazolyl group, a thiadiazolyl group, a triazolyl group, a tetrazolyl group, a pyridyl group, a pyrimidyl group, a pyrazinyl group, a pyridazinyl group, a benzofuranyl group, an isobenzofuranyl group, a benzothienyl group, an indolyl group, an isoindolyl group, an indazolyl group, a benzimidazolyl group, a benzoxazolyl group, a benzisoxazolyl group, a benzothiazolyl group, a benzisothiazolyl group, a benzoxadiazolyl group, a benzothiadiazolyl group, a benzotriazolyl group, a quinolyl group, an isoquinolyl group, a cinnolinyl group, a quinazolinyl group, a quinoxalinyl group, a phthalazinyl group, a naphthyridinyl group, a purinyl group, a pteridinyl group, a furopyridyl group, a thienopyridyl group, a pyrrolopyridyl group, an oxazolopyridyl group, a thiazolopyridyl group, and an imidazopyridyl group, and the thienyl group is preferable.

In the specification, examples of the "substituent" in the "optionally substituted $C_6$-$C_{10}$ aryl group" and "optionally substituted 5 to 10-membered heteroaryl group" specifically include a halogen atom and -$CH_2$-OC(=O)-$R_5$ (wherein, $R_5$ means a $C_6$-$C_{10}$ aryl group optionally having one to three $C_1$-$C_4$ alkyl group (s) as substituent(s) (e.g., 2,4-dimethylphenyl group), and a chlorine atom and a 2,4-dimethylphenyl carboxymethyl group are preferable.

In the specification, it is preferred that the "optionally substituted $C_6$-$C_{10}$ aryl group" is a 4-chlorophenyl group and a 4-(2,4-dimethylphenyl carboxymethyl)phenyl group.

In the specification, it is preferred that the "optionally substituted 5 to 10-membered heteroaryl group" is a 3-thienyl group.

When $R_3$ is a hydroxy group, the substitution position of $R_3$ is not limited as long as it is on the isoquinoline ring, but it is preferably position 1 of the isoquinoline ring.

In the specification, the "compound represented by the formula (1) or a salt thereof or a solvate thereof" is preferably a compound represented by the following formula (2):

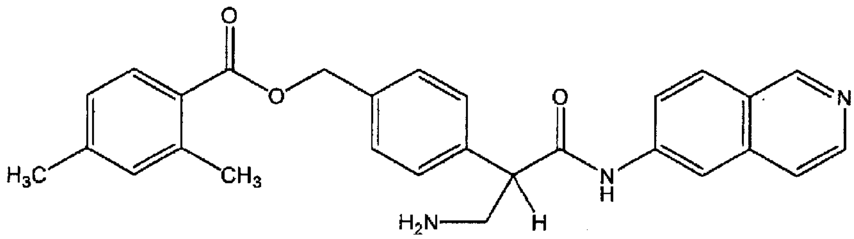

(2)

or a salt thereof or a solvate thereof; more preferably a compound represented by the following formula (3):

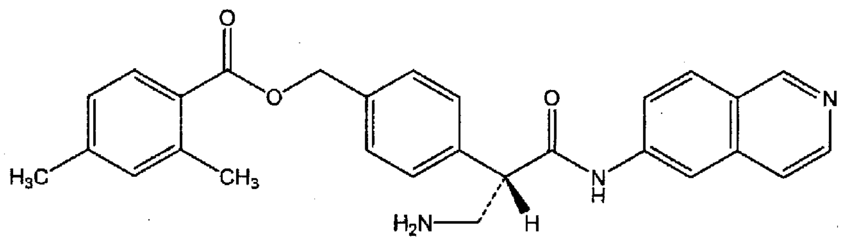

(3)

(netarsudil) (Chemical name: [4-[(2S)-3-amino-1-(isoquinolin-6-ylamino)-1-oxopropan-2-yl]phenyl]methyl 2,4-dimethylbenzoate), International Nonproprietary Name: netarsudil) or a salt thereof or a solvate thereof; and particularly preferably a compound represented by the following formula (4):

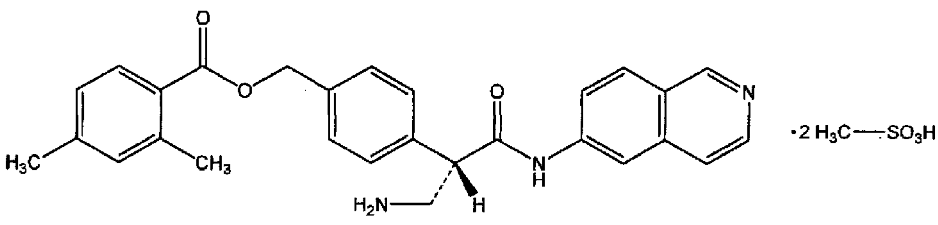

(4)

, which is a dimethanesulfonate of the compound represented by the formula (3), (chemical name: [4-[(2S)-3-amino-1-(isoquinolin-6-ylamino)-1-oxopropan-2-yl]phenyl]methyl 2,4-dimethylbenzoate dimethanesulfonate), hereinafter in the specification, it is sometimes referred to as "netarsudil dimesylate").

As another embodiment, the "compound represented by the formula (1) or a salt thereof or a solvate thereof" is preferably a compound represented by the following formula (5) or (5'):

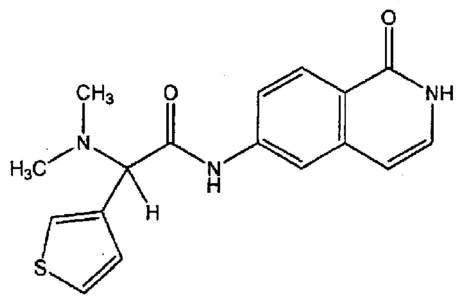

(5)

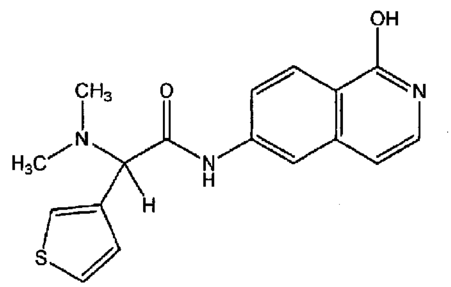

(5')

or a salt thereof or a solvate thereof; more preferably a compound represented by the following formula (6):

rac 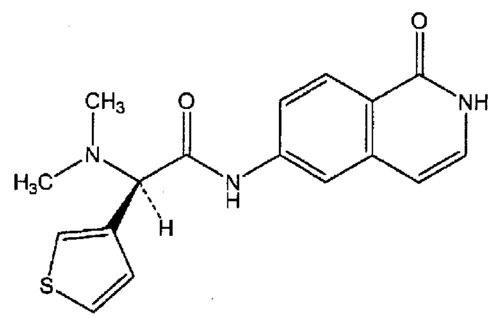

(6)

(verosudil) (Chemical name: rac-(2R)-2-(dimethylamino)-N-(1-oxo-1,2-dihydroisoquinolin-6-yl)-2-(thiophen-3-yl)acetamide), International Nonproprietary Name: verosudil), or a tautomer thereof or a salt of these or a solvate thereof; and particularly preferably a compound represented by the following formula (7):

rac 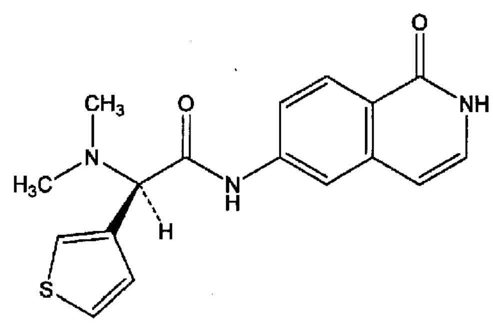 •HCl

(7)

, which is a monohydrochloride of the compound represented by the formula (6), (chemical name: rac-(2R)-2-(dimethylamino)-N-(1-oxo-1,2-dihydroisoquinolin-6-yl)-2-(thiophen-3-yl)acetamide monohydrochloride, hereinafter in the specification, it is sometimes referred to as "verosudil monohydrochloride").

Further, as another embodiment, the "compound represented by the formula (1) or a salt thereof or a solvate thereof" is preferably a compound represented by the following formula (8):

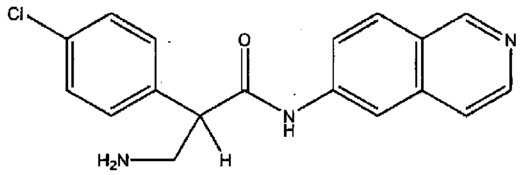

(8)

(3-amino-2-(4-chlorophenyl)-N-(isoquinolin-6-yl)propanamide) or a salt thereof or a solvate thereof.

The compound represented by the formula (1) or a salt thereof or a solvate thereof is known compounds, and they can be prepared by a known method. Specifically, for example, the compounds can be prepared with reference to the methods described in Patent Literatures 1 to 3 or Non Patent Literature 2. The contents of these literatures are incorporated herein by reference.

The compound represented by the formula (1) or a salt thereof or a solvate thereof is commercially available, and these commercial products may be used. Specific examples of the commercial products include netarsudil dimesylate (the compound represented by the formula (4)) manufactured by MedChemExpress and by Chemscene LLS; netarsudil monohydrochloride (monohydrochloride of the compound represented by the formula (3)) manufactured by Shanghai Biopharmaleader Co., Ltd.; and verosudil (a free form of the compound represented by the formula (6)) manufactured by MedKoo Biosciences, Inc.

The content of the compound represented by the formula (1) or a salt thereof or a solvate thereof in the aqueous composition is not particularly limited, and can be appropriately determined depending on the applicable disease and patient's sex, age, symptoms or the like, but, from the viewpoint of obtaining an excellent pharmacological action, the content is preferably from 0.0001 to 5 w/v%, more preferably from 0.001 to 3 w/v%, still more preferably from 0.005 to 2 w/v %, and particularly preferably from 0.01 to 1 w/v% of the compound represented by the formula (1) in terms of a free form relative to the total volume of the aqueous composition.

Particularly, when netarsudil is used as the compound represented by the formula (1), the content is preferably from 0.0001 to 1 w/v%, more preferably from 0.001 to 0.5 w/v%, still more preferably from 0.005 to 0.1 w/v%, and particularly preferably from 0.01 to 0.04 w/v% of netarsudil in terms of a free form relative to the total volume of the aqueous composition, from the viewpoint of obtaining excellent pharmacological action.

Further, when verosudil is used as the compound represented by the formula (1), from the viewpoint of obtaining excellent pharmacological action, the content is preferably from 0.01 to 3.5 w/v%, more preferably from 0.1 to 2.5 w/v%, still more preferably from 0.2 to 1.5 w/v%, and particularly preferably from 0.3 to 0.8 w/v% of verosudil in terms of a free form relative to the total volume of the aqueous composition.

Furthermore, when the compound represented by the formula (8) is used as the compound represented by the formula (1), the content is preferably from 0.0001 to 3 w/v%, more preferably from 0.001 to 2 w/v%, still more preferably from 0.005 to 1 w/v%, and particularly preferably from 0.01 to 0.5 w/v% of the compound represented by the formula (8) in terms of a free form relative to the total volume of the aqueous composition, from the viewpoint of obtaining excellent pharmacological action.

In the present specification, the "aqueous composition" means a composition comprising at least water, and the property of the aqueous composition is not particularly limited as long as it can be housed in a container described below, and for example, the aqueous composition may be in the form of liquid (solution or suspension), semi-solid (ointment), and the like. Examples of the water in the composition include purified water, water for injection, and sterilized purified water.

The content of water in the aqueous composition is not particularly limited, but preferably 5 % by mass or more, more preferably 20 % by mass or more, still more preferably 50 % by mass or more, even more preferably 90 % by mass or more, and particularly preferably from 90 to 99.99 % by mass.

The aqueous composition may comprise one or more additives utilized in pharmaceutical products, quasi-drugs and the like, besides the components described above, depending on the dosage form. Examples of the additives include an inorganic salt, an isotonic agent, a chelating agent, a stabilizing agent, a pH adjusting agent, a preservative, an antioxidant, a thickening agent, a surfactant, a solubilizer, a suspending agent, a refreshing agent, a dispersing agent, a preserving agent, an oily base, an emulsion base, and a water-soluble base.

Specific examples of the additive include ascorbic acid, potassium aspartate, sodium bisulfite, alginic acid, sodium benzoate, benzyl benzoate, epsilon-aminocaproic acid, fennel oil, ethanol, an ethylene-vinyl acetate copolymer,

sodium edetate, tetrasodium edetate, potassium chloride, calcium chloride hydrate, sodium chloride, magnesium chloride, hydrochloric acid, alkyldiaminoethylglycine hydrochloride solution, a carboxyvinyl polymer, dried sodium sulfite, dried sodium carbonate, d-camphor, dl-camphor, xylitol, citric acid hydrate, sodium citrate hydrate, glycerin, gluconic acid, L-glutamic acid, sodium L-glutamate, creatinine, chlorhexidine gluconate solution, chlorobutanol, crystalline sodium dihydrogen phosphate dihydrate, geraniol, sodium chondroitin sulfate, acetic acid, potassium acetate, sodium acetate hydrate, titanium oxide, gellan gum, dibutylhydroxytoluene, potassium bromide, benzododecinium bromide, tartaric acid, sodium hydroxide, polyoxyl 45 stearate, purified lanolin, D-sorbitol, sorbitol solution, sorbic acid, potassium sorbate, taurine, sodium hydrogen carbonate, sodium carbonate hydrate, sodium thiosulfate hydrate, thimerosal, tyloxapol, sodium dehydroacetate, trometamol, concentrated glycerin, a concentrated mixed tocopherol, white petrolatum, peppermint water, peppermint oil, concentrated benzalkonium chloride solution 50, ethyl parahydroxybenzoate, butyl parahydroxybenzoate, propyl parahydroxybenzoate, methyl parahydroxybenzoate, sodium hyaluronate, human serum albumin, hydroxyethyl cellulose, hydroxypropyl cellulose, hypromellose, glacial acetic acid, sodium pyrosulfite, phenyl ethyl alcohol, glucose, propylene glycol, bergamot oil, benzalkonium chloride, benzalkonium chloride solution, benzyl alcohol, benzethonium chloride, benzethonium chloride solution, borax, boric acid or a salt thereof (e.g., boric acid, ammonium borate, borax), povidone, polyoxyethylene (200) polyoxypropylene glycol (70), sodium polystyrenesulfonate, polysorbate 80, polyoxyethylene hydrogenated castor oil 60, polyvinyl alcohol (partially saponified product), d-borneol, macrogol 4000, macrogol 6000, D-mannitol, anhydrous citric acid, disodium hydrogen phosphate anhydrous, sodium dihydrogen phosphate anhydrous, methanesulfonic acid, methyl cellulose, 1-menthol, monoethanolamine, aluminum monostearate, polyethylene glycol monostearate, eucalyptus oil, potassium iodide, sulfuric acid, oxyquinoline sulfate, liquid paraffin, borneol, phosphoric acid, dibasic sodium phosphate, monobasic potassium phosphate, sodium dihydrogen phosphate dihydrate, sodium dihydrogen phosphate monohydrate, malic acid, and vaseline.

Among the above, for example, potassium chloride, calcium chloride hydrate, sodium chloride, magnesium chloride, glycerin, acetic acid, potassium acetate, sodium acetate hydrate, tartaric acid, sodium hydroxide, sodium hydrogen carbonate, sodium carbonate hydrate, concentrated glycerin, hydroxyethyl cellulose, hydroxypropyl cellulose, hypromellose, borax, boric acid or a salt thereof, povidone, polysorbate 80, polyoxyethylene hydrogenated castor oil, polyethylene glycol monostearate, polyvinyl alcohol (partially saponified product), macrogol 4000, macrogol 6000, anhydrous citric acid, disodium hydrogen phosphate anhydrous, sodium dihydrogen phosphate anhydrous, methyl cellulose, monoethanolamine, phosphoric acid, dibasic sodium phosphate, monobasic potassium phosphate, sodium dihydrogen phosphate dihydrate, sodium dihydrogen phosphate monohydrate, sodium hyaluronate, glucose, or 1-menthol is preferred as the additive.

The aqueous composition may further comprise, in addition to the components described above, one or more other medicinal components, depending on the applicable disease or the like. Examples of the medicinal components include α1 receptor blockers including bunazosin or a salt thereof or a solvate thereof such as bunazosin hydrochloride; α2 receptor agonists including brimonidine or a salt thereof or a solvate thereof such as brimonidine tartrate, and apraclonidine or a salt thereof or a solvate thereof; β-blockers including carteolol or a salt thereof or a solvate thereof such as carteolol hydrochloride, nipradilol or a salt thereof or a solvate thereof, timolol or a salt thereof or a solvate thereof such as timolol maleate, betaxolol or a salt thereof or a solvate thereof such as betaxolol hydrochloride, levobunolol or a salt thereof or a solvate thereof such as levobunolol hydrochloride, befunolol or a salt thereof or a solvate thereof, and metipranolol or a salt thereof or a solvate thereof; carbonic anhydrase inhibitors including dorzolamide or a salt thereof or a solvate thereof such as dorzolamide hydrochloride, brinzolamide or a salt thereof or a solvate thereof, acetazolamide or a salt thereof or a solvate thereof, dichlorphenamide or a salt thereof or a solvate thereof, and methazolamide or a salt thereof or a solvate thereof; prostaglandins and their derivatives (e.g., prostaglandin F2α derivatives) including isopropyl unoprostone or a salt thereof or a solvate thereof, tafluprost or a salt thereof or a solvate thereof, travoprost or a salt thereof or a solvate thereof, bimatoprost or a salt thereof or a solvate thereof, latanoprost or a salt thereof or a solvate thereof, cloprostenol or a salt thereof or a solvate thereof, and fluprostenol or a salt thereof or a solvate thereof; Rho kinase inhibitors including Ripasudil or a salt thereof or a solvate thereof, Y-39983 and H-1129; sympathomimetic drugs including dipivefrine or a salt thereof or a solvate thereof such as dipivefrin hydrochloride, and epinephrine or a salt thereof or a solvate thereof such as epinephrine, epinephrine borate, or epinephrine hydrochloride; parasympathomimetic drugs including distigmine bromide or a salt thereof or a solvate thereof, pilocarpine or a salt thereof or a solvate thereof such as pilocarpine, pilocarpine hydrochloride or pilocarpine nitrate, and carbachol or a salt thereof or a solvate thereof; calcium antagonists including lomerizine or a salt thereof or a solvate thereof such as lomerizine hydrochloride; and cholinesterase inhibitors including demecarium or a salt thereof or a solvate thereof, echothiophate or a salt thereof or a solvate thereof, and physostigmine or a salt thereof or a solvate thereof. These medicinal components can be blended singly or a combination of two or more.

As the other medicinal components, β-blockers are preferred, and especially timolol is preferred.

The aqueous composition may be one which does not contain the prostaglandin and any derivative thereof. For example, one embodiment of the aqueous composition is preferably other than the following <A-1> to <A-10>:

<A-1> a composition comprising (rac)-2-(dimethylamino)-N-(1-hydroxy-isoquinolin-6-yl)-2-(thiophen-3-yl) acetamide hydrochloride, travoprost, boric acid, D-mannitol, benzalkonium chloride, polyoxyl 40 stearate, polyethylene glycol 400, EDTA, and purified water.
<A-2> a composition comprising (rac)-2-(dimethylamino)-N-(1-hydroxy-isoquinolin-6-yl)-2-(thiophen-3-yl) acetamide hydrochloride, travoprost, boric acid, D-mannitol, benzalkonium chloride, Cremophor RH40, polyethylene glycol 400, EDTA, and purified water.
<A-3> a composition comprising (rac)-2-(dimethylamino)-N-(1-hydroxy-isoquinolin-6-yl)-2-(thiophen-3-yl)acetamide hydrochloride, travoprost, boric acid, D-mannitol, polyoxyl 40 stearate, polyethylene glycol 400, EDTA, and purified water.
<A-4> a composition comprising (rac)-2-(dimethylamino)-N-(1-hydroxy-isoquinolin-6-yl)-2-(thiophen-3-yl)acetamide hydrochloride, latanoprost, monobasic sodium phosphate, dibasic sodium phosphate, benzalkonium chloride, sodium chloride, EDTA, and purified water.
<A-5> a composition comprising (rac)-2-(dimethylamino)-N-(1-hydroxy-isoquinolin-6-yl)-2-(thiophen-3-yl)acetamide hydrochloride, latanoprost, boric acid, D-mannitol, benzalkonium chloride, EDTA, and purified water.
<A-6> a composition comprising (rac)-2-(dimethylamino)-N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.
<A-7> a composition comprising (rac)-2-(dimethylamino)-N-(1-hydroxy-isoquinolin-6-yl)-2-(thiophen-3-yl)acetamide hydrochloride, bimatoprost, monobasic sodium phosphate, dibasic sodium phosphate, benzalkonium chloride, sodium chloride, EDTA, and purified water.
<A-8> a composition comprising (rac)-2-(dimethylamino)-N-(1-hydroxy-isoquinolin-6-yl)-2-(thiophen-3-yl)acetamide hydrochloride, bimatoprost, boric acid, D-mannitol, and purified water.
<A-9> a composition comprising (S)-4-(3-amino-1-(isoquinolin-6-ylamino)-1-oxopropan-2-yl)benzyl 2,4-dimethylbenzoate, travoprost, boric acid, D-mannitol, benzalkonium chloride, polyoxyl 40 stearate, polyethylene glycol 400, EDTA, and purified water.
<A-10> a composition comprising (S)-4-(3- amino-1-(isoquinolin-6-ylamino)-1-oxopropan-2-yl)benzyl 2,4-dimethylbenzoate, latanoprost, boric acid, D-mannitol, benzalkonium chloride, polyoxyl 40 stearate, polyethylene glycol 400, EDTA, and purified water.

The pH of the aqueous composition (at 25°C) is not particularly limited, but preferably from 3 to 9, more preferably from 3.5 to 8, still more preferably from 4 to 7, particularly preferably from 5 to 6. Further, the osmotic pressure ratio of the aqueous composition to physiological saline is not particularly limited, but preferably from 0.6 to 3, particularly preferably from 0.6 to 2.

The pharmaceutical preparation of the present invention uses a container formed of a polyester resin or a polyolefin resin.

In the specification, the "container" means a package for directly housing the aqueous composition. The concept of the container encompasses any of "well-closed container", "tight container" and "hermetic container" which are defined in the Japanese Pharmacopoeia, Seventeenth Edition, General Notices.

The form of the container is not particularly limited as long as it is capable of housing the aqueous composition, and may be appropriately selected and set depending on the dosage form, the application of the pharmaceutical preparation, and the like. Specific examples of the container in such a form include a container for injection, a container for inhalation, a container for spray, a bottle-shaped container, a tube-shaped container, an eye drop container, a nasal drop container, an ear drop container, a bag shaped container, and the like.

The container, from the viewpoint of advantageously utilizing pharmacological action of the compound represented by the formula (1), is preferably an eye drop container.

In the specification, the "container formed of a polyester resin" means a container of which at least a part in contact with the aqueous composition is "formed of a polyester resin". Thus, for example, a container having a polyester resin layer as an inner layer in contact with an aqueous composition, and further having a layer of other resin material laminated on the outside of the inner layer also corresponds to the "container formed of a polyester resin". Here, dicarboxylic acid and diol which constitute the polyester resin are not particularly limited, and examples of the dicarboxylic acids include phthalic acid, terephthalic acid and 2,6-naphthalene dicarboxylic acid, and examples of the diol include ethylene glycol, 1,3-propanediol, 1,4-butanediol, 1,4-cyclohexanedimethanol, and bisphenol. The polyester resin may be a polymer composed of single kind of polyester unit or a polymer composed of plural kinds of polyester units. In the case of a polymer composed of plural kinds of polyester units, the polymerization mode is not particularly limited and may be a random polymerization or a block polymerization. Furthermore, the stereoregularity (tacticity) thereof is not particularly limited.

Specific examples of the polyester resin include homopolyester such as polyalkylene terephthalate (e.g., polyethylene terephthalate, polybutylene terephthalate), polyalkylene naphthalate (e.g., polyethylene naphthalate, polybutylene naphthalate), polycycloalkylene terephthalate (e.g., poly(1,4-cyclohexylene dimethylene terephthalate) or polyarylate (e.g., resin composed of bisphenol and phthalic acid); copolyester comprising units of the homopolyester as the main component; and a copolymer of the homopolyester. These resins can be used singly or in a combination of two or more. From the viewpoint of suppressing the content reduction of the compound represented by the formula (1) at the time of storage at high temperature, the polyester resin is preferably, polyethylene terephthalate.

In the specification, the phrase "formed of a polyester resin" means that the material comprises at least in part a polyester resin. For example, those formed of a mixture of two or more kinds of resin, a polyester resin and one or more other kind(s) of resin, (polymer alloy) also corresponds to those "formed of a polyester resin".

In the specification, the "container formed of a polyolefin resin" means a container of which at least a part in contact with the aqueous composition is "formed of a polyolefin resin". Thus, for example, a container having a polyolefin resin layer as an inner layer in contact with an aqueous composition, and further having a layer of other resin material laminated on the outside of the inner layer also corresponds to the "polyolefin resin container". The polyolefin resin is not particularly limited, and it may be a polymer composed of single kind of monomer (homopolymer) or a copolymer composed of plural kinds of monomers (copolymer). In the case of a copolymer, the polymerization mode is not particularly limited, and the copolymer may be a random copolymer or a block copolymer. Furthermore, the stereoregularity (tacticity) of the copolymer is not particularly limited.

Specific examples of the polyolefin resin include polyethylene (more specifically, including, low density polyethylene (linear low density polyethylene), high density polyethylene, medium density polyethylene), polypropylene, cyclic polyolefin, poly(4-methylpentene), polytetrafluoroethylene, an ethylene-propylene copolymer, an ethylene-$\alpha$-olefin copolymer, an ethylene-acrylic acid copolymer, an ethylene-methacrylic acid copolymer, an ethylene-vinyl acetate copolymer, and an ethylene-ethyl acrylate copolymer. These resins can be used singly or in a combination of two or more. From the viewpoint of suppressing the content reduction of the compound represented by the formula (1) due to storage under high temperature, the polyolefin resin is preferably, polyethylene, polypropylene, or cyclic polyolefin, more preferably, low density polyethylene, medium density polyethylene, high density polyethylene, or polypropylene, and particularly preferably, low density polyethylene, high density polyethylene, or polypropylene.

As used herein, the phrase "formed of a polyolefin resin" means that the material comprises, at least in part, a polyolefin resin. For example, the one formed of a mixture of two or more kinds of resin (polymer alloy) of a polyolefin resin and one or more other kinds of resin also correspond to the one "formed of a polyolefin resin".

It is preferred that a substance which interferes with the transmission of ultraviolet light, such as an ultraviolet absorbing agent or an ultraviolet scattering agent is kneaded in a container. With the container in which such a substance has been kneaded, the light stability of the compound represented by the formula (1) is improved. Specific examples of the ultraviolet scattering agent include titanium oxide and zinc oxide. Examples of the ultraviolet absorbing agent include: benzotriazole-based ultraviolet absorbent such as 2-(2H-benzotriazol-2-yl)-p-cresol (e.g., Tinuvin P: BASF), 2-(2H-benzotriazol-2-yl)-4,6-bis(1-methyl-1-phenylethyl)phenol (e.g., Tinuvin 234: BASF), 2-(3,5-di-tert-butyl-2-hydroxyphenyl)benzotriazole (e.g., Tinuvin 320: BASF), 2-[5-chloro-(2H)-benzotriazol-2-yl]-4-methyl-6-t-butyl-phenol (e.g., Tinuvin 326: BASF), 2-(3,5-di-t-butyl-2-hydroxyphenyl)-5-chlorobenzotriazole (e.g., Tinuvin 327: BASF), 2-(2H-benzotriazol-2-yl)-4,6-di-tert-pentylphenol (e.g., Tinuvin PA328: BASF), 2-(2H-benzotriazol-2-yl)-4-(1,1,3,3-tetramethylbutyl)phenol (e.g., Tinuvin 329: BASF), 2,2'-methylenebis [6-(2H-benzotriazol-2-yl)-4-(1,1,3,3-tetramethylbutyl)phenol (e.g., Tinuvin 360: BASF), a reaction product of methyl 3-(3-(2H-benzotriazol-2-yl)-5-tert-butyl-4-hydroxyphenyl)propionate and polyethylene glycol 300 (e.g., Tinuvin 213: BASF), 2-(2H-benzotriazol-2-yl)-6-dodecyl-4-methylphenol (e.g., Tinuvin 571: BASF), 2-(2'-hydroxy-3',5'-di-t-amylphenyl)benzotriazole, 2-[2'-hydroxy-3'-(3",4",5",6"-tetrahydrophthalimidemethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbing agent such as 2,2-bis{[2-cyano-3,3-diphenyl acryloyloxylmethyllpropane-1,3-diyl=bis(2-cyano-3,3-diphenylacrylate) (for example, Uvinul 3030 FF: BASF), ethyl 2-cyano-3,3-diphenyl acrylate (e.g., Uvinul 3035: BASF), and 2-ethylhexyl 2-cyano-3,3-diphenylacrylate (e.g., Uvinul 3039: BASF) and others; triazine-based ultraviolet absorbing agent such as 2-(4,6-diphenyl-1,3,5-triazin-2-yl)-5-[(hexyl)oxy]-phenol (e.g., Tinuvin 1577 ED: BASF); benzophenone-based ultraviolet absorbing agent such as octabenzone (e.g., Chimassorb 81: BASF), 2,2'-dihydroxy-4,4'-dimethoxy benzophenone (e.g., Uvinul 3049: BASF), 2,2'-4,4'-tetrahydrobenzophenone (e.g., Uvinul 3050: BASF), oxybenzone, hydroxymethoxybenzophenone sulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamic acid-based ultraviolet absorbing agent such as methyl diisopropylcinnamate, cinoxate, glyceryl mono-2-ethylhexanoate diparamethoxycinnamate, a mixture of isopropyl paramethoxycinnamate and diisopropyl cinnamate, 2-ethylhexyl paramethoxycinnamate, and benzyl cinnamate; benzoic acid ester-based ultraviolet absorbing agent such as para-aminobenzoic acid, ethyl para-aminobenzoate, glyceryl para-aminobenzoate, amyl para-dimethylaminobenzoate, 2-ethylhexyl para-dimethylaminobenzoate, and ethyl 4-[N,N-di(2-hydroxypropyl) amino]benzoate; salicylic acid-based ultraviolet absorbing agent such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino] 1,3,5-triazine; para-hydroxy anisole; 4-tert-butyl-4'-methoxydibenzoylmethane; phenylbenzimidazole sulfonate; and hexyl 2-(4-diethylamino-2-hydroxybenzoyl) benzoate.

When a substance which interferes with the transmission of ultraviolet light is kneaded in the container, the blending ratio is determined depending on the kinds of the substance and the like, but, for example, the ratio of the substance in the container may be about from 0.001 to 50 % by mass, preferably from 0.002 to 25 % by mass, particularly preferably from 0.01 to 10 % by mass.

It is preferred that the inside of the container is visible (observable) to the naked eye or the like. When the inside is visible, there are some advantages that it becomes possible to examine the presence or absence of contamination or the like in the manufacturing process of the pharmaceutical preparation; and that the user of the pharmaceutical preparation can check the remained content (aqueous composition), and the like. Here, it is sufficient if the visibility is ensured in at least a portion of the surface of the container (For example, an eye drop container having a bottom surface allowing the inside of the container to be visible corresponds to the container which is visible, even when the inside of the container is not visible from the side surface owing to a shrink film or the like). When the inside of the container is visible from at least a portion of the surface of the container, it is possible to confirm the aqueous composition inside of the container.

The means for housing the aqueous composition in the container is not particularly limited and it can be performed by filling or the like with an ordinary method depending on the form of the container or the like.

The pharmaceutical preparation or aqueous composition can be formulated in various dosage forms according to a known method described in the Japanese Pharmacopoeia, Seventeenth Edition, General Rules for Preparation or the like. Examples of the dosage form include injection, inhalation liquid, eye drop, eye ointment, ear drop, nasal drop liquid, enema formulation, topical liquid, spray, ointment, gel, oral liquid, and syrup. From the viewpoint of advantageously utilizing the pharmacological actions of the compound represented by the formula (1), the dosage form is preferably, a dosage form for eye disease, specifically, eye drop or eye ointment, and particularly preferably, eye drop.

The applicable disease of the pharmaceutical preparation is not particularly limited, and it is appropriately selected depending on the pharmacological actions of the compound represented by the formula (1) or the like.

Specifically, for example, the pharmaceutical preparation can be used as a prevention or treatment agent for ocular hypertension and glaucoma, based on Rho kinase inhibitory activity, norepinephrine transporter inhibitory activity, and intraocular pressure-decreasing activity of the compound represented by the formula (1). Detailed examples of the glaucoma include primary open-angle glaucoma, normal tension glaucoma, hypersecretion glaucoma, acute angle closure glaucoma, chronic angle closure glaucoma, plateau iris syndrome, mixed-type glaucoma, steroid glaucoma, glaucoma capsulare, pigment glaucoma, amyloid glaucoma, angiogenesis glaucoma, and malignant glaucoma.

In the case of using the aqueous composition or pharmaceutical preparation as a prevention or treatment agent for eye disease (appropriately, a diseases selected from the group of ocular hypertension and glaucoma), the aqueous composition or pharmaceutical preparation may be administered, for example, about 1 to 3 times a day in a suitable dose.

Hereinafter, examples of embodiments or aspects of the present invention are disclosed below; however, the present invention is not limited thereto.

[1] A pharmaceutical preparation comprising:
an aqueous composition comprising a compound represented by the following formula (1)

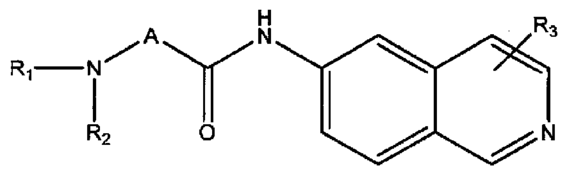

(1)

(in the formula, $R_1$ and $R_2$ each independently represent a hydrogen atom or a $C_1$-$C_4$ alkyl group,
$R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof)
or a salt thereof or a solvate thereof, the aqueous composition being housed in a container formed of a polyester resin or a polyolefin resin.

[2] The pharmaceutical preparation according to [1], wherein the compound represented by the formula (1) is a compound represented by the following formula (2), (5), (5') or (8) :

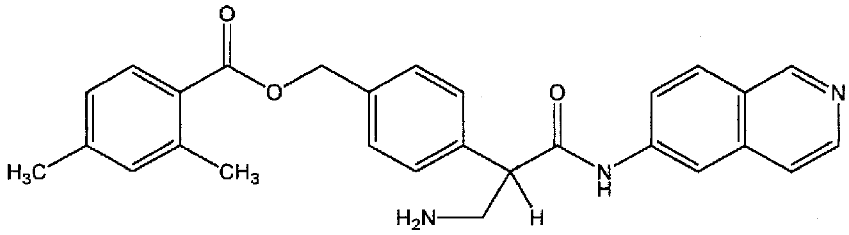

(2) ;

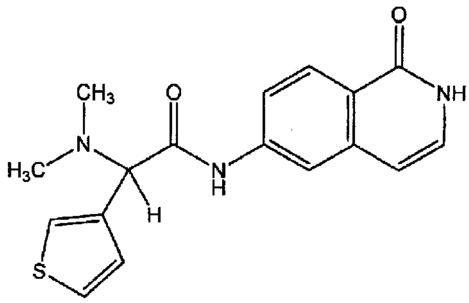

(5) ;

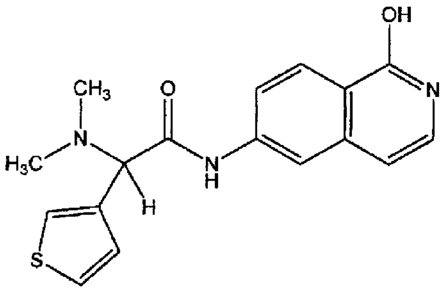

(5') ;

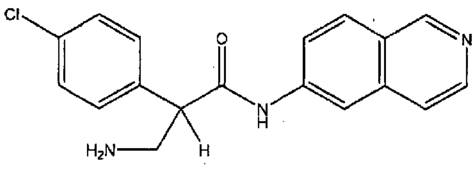

(8) .

[3] The pharmaceutical preparation according to [1], wherein the compound represented by the formula (1) is a compound represented by the following formula (3) or (6):

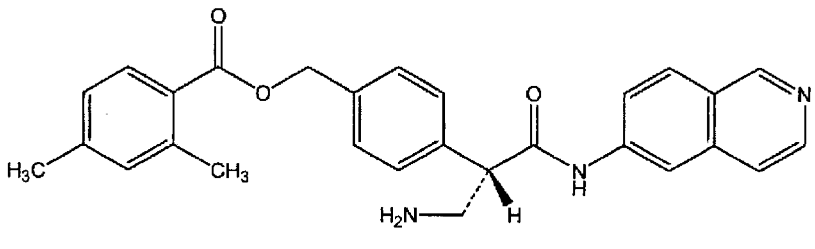

(3) ;

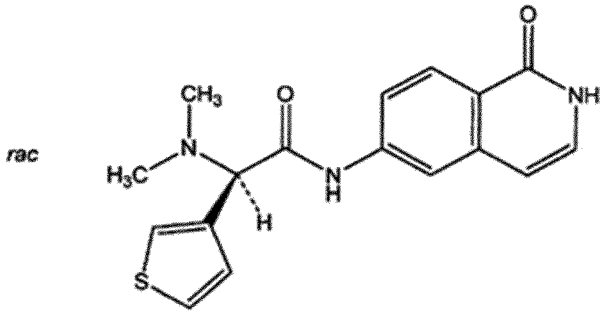

(6) .

[4] The pharmaceutical preparation according to [1], wherein the compound represented by the formula (1) is a compound represented by the following formula (4) or (7):

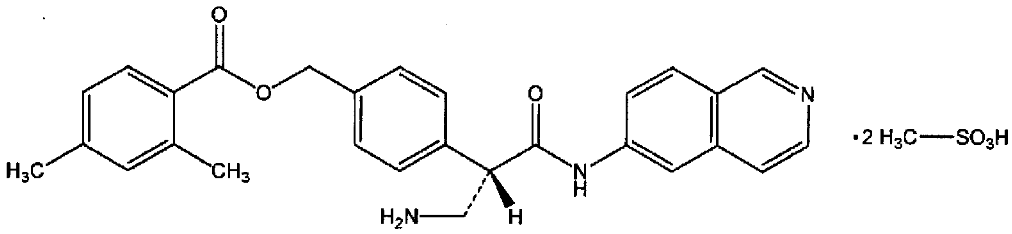

(4) ;

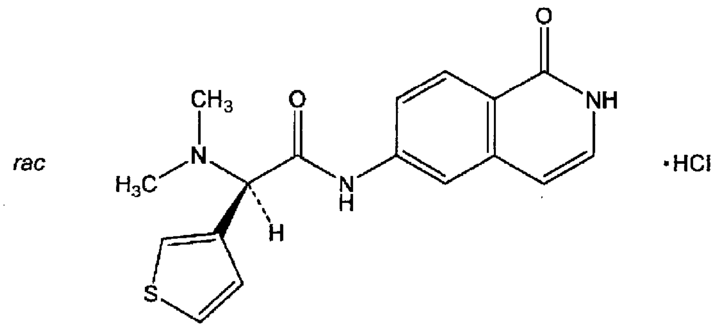

(7) .

[5] The pharmaceutical preparation according to any one of [1] to [4], wherein the polyester resin is polyethylene terephthalate.

[6] The pharmaceutical preparation according to any one of [1] to [5], wherein the polyolefin resin is polyethylene or polypropylene.

[7] The pharmaceutical preparation according to any one of [1] to [6], wherein the container is a container for an ophthalmic solution.

[8] A method of improving heat stability of a compound represented by the formula (1) or a salt thereof or a solvate thereof in an aqueous composition, comprising a step of housing the aqueous composition comprising the compound represented by the formula (1) or a salt thereof or a solvate thereof in a container formed of a polyester resin or a polyolefin resin. (Here, the "method of improving heat stability" means a method of suppressing the content reduction at the time of storage at a high temperature. For example, when the content reduction in the aqueous composition after storage at a high temperature (e.g., 80°C) for a certain period (e.g., 1 week) is suppressed by the method relative to that in the same composition housed in a container formed of glass, it is considered that the method "improves heat stability".)

[9] The method according to [8], wherein the compound represented by the formula (1) is a compound represented by the formula (2), (5), (5') or (8).

[10] The method according to [8], wherein the compound represented by the formula (1) is a compound represented by the formula (3) or (6).

[11] The method according to [8], wherein the compound represented by the formula (1) is a compound represented by the formula (4) or (7).

[12] The method according to any one of [8] to [11], wherein the polyester resin is polyethylene terephthalate.
[13] The method according to any one of [8] to [12], wherein the polyolefin resin is polyethylene or polypropylene.
[14] The method according to any one of [8] to [13], wherein the container is a container for an ophthalmic solution.

## Examples

Next, the present invention is further described by way of Examples, but the present invention is not limited to these Examples.

In the following Test Examples, the measurements of netarsudil using HPLC were performed using an ODS column as the column, 0.01 mol/L phosphate buffer solution and acetonitrile as the mobile phase, and a UV absorptiometer as the detector (wavelength: 254 nm), respectively.

### [Test Example 1] Heat Stability Test

The heat stability of netarsudil in the aqueous composition was assessed by examining the presence or absence of content reduction of netarsudil after stored under high temperature for a certain period.

In other words, an aqueous composition having the formulation shown in Table 1 was prepared with an ordinary method, and then the obtained composition was housed in a container formed of polyethylene terephthalate (PET), low-density polyethylene (LDPE), high density polyethylene (HDPE) or polypropylene (PP) to obtain pharmaceutical preparations of Examples 1 to 4, respectively. In addition, the same aqueous composition was housed in a container formed of glass to obtain a pharmaceutical preparation of Comparative Example.

Each of the obtained pharmaceutical preparations was stored at 80°C for a week. To examine the presence or absence of content reduction of netarsudil after storage, the concentrations of netarsudil in the aqueous composition before and after storage were measured for each of the pharmaceutical preparation. The concentration of netarsudil in the aqueous composition was calculated by measuring the ratio of the peak area of the aqueous composition relative to the peak area of a netarsudil solution of a known concentration, using HPLC.

Then, based on the calculated concentration of netarsudil in the aqueous composition, the residual rate (%) of netarsudil was assessed using the following expression:

$$\text{Residual rate (\%)} = \{(\text{Concentration of netarsudil in the aqueous composition after storage})/(\text{Concentration of netarsudil in the aqueous composition before storage})\} \times 100$$

The results are shown in Table 2.

[Table 1]

| Component | Amount (per 100 mL) |
|---|---|
| Netarsudil dimesylate | 0.02848 g (0.02 g in terms of a free form) |
| Boric acid | 0.05 g |
| Sodium hydroxide | q.s. (pH 5.0) |
| Purified water | Balance |

[Table 2]

| | Class of pharmaceutical preparation and material of container | After storage at 80°C for a week |
|---|---|---|
| Residual rate (%) | Example 1: PET | 75.1 |
| | Example 2: LDPE | 72.1 |
| | Example 3: HDPE | 71.4 |
| | Example 4: PP | 70.7 |
| | Comparative Example: glass | 47.5 |

As shown in the results listed in Table 2, when the aqueous composition comprising netarsudil was housed in the container formed of polyethylene terephthalate (PET) (Examples 1), the container formed of polyethylene (LDPE, HDPE) (Examples 2, 3) or the container formed of polypropylene (PP) (Example 4), the content reduction due to storage under high temperature was suppressed relative to the case housed in a container formed of glass (Comparative Example).

In particular, when the aqueous composition was housed in a container formed of polyethylene terephthalate (PET) or polyethylene (LDPE, HDPE), the degree of suppression of the content reduction was significant.

From the results of Test Example 1, it was revealed that, by housing the aqueous composition comprising the compound represented by the formula (1) exemplified by netarsudil or a salt thereof or a solvate thereof in a container formed of a polyester resin exemplified by polyethylene terephthalate or a container formed of a polyolefin resin exemplified by polyethylene or polypropylene, the content reduction is relatively suppressed even when the aqueous composition is stored under high temperature conditions, and a pharmaceutical preparation having excellent storage stability is obtained.

[Test Example 2] Adsorption Test

To investigate the presence or absence of adsorption of netarsudil to the container formed of a polyester resin, a piece formed of a polyester resin was placed in an aqueous composition comprising netarsudil and stored for a certain period of time and then the presence or absence of the content reduction of netarsudil in the aqueous composition was examined.

In other words, aqueous compositions shown in Table 3 were prepared with an ordinary method, and 5 mL of the obtained composition each was housed in a glass container, and then, into the aqueous composition, three resin pieces (each having a size of 1 cm x 2 cm) formed of polyethylene terephthalate (PET) were immersed to obtain a pharmaceutical preparation of Reference Example 1. In addition, a pharmaceutical preparation was prepared in the same way as Reference Example 1 except for not immersing resin pieces formed of polyethylene terephthalate to obtain a pharmaceutical preparation of Reference Example 2.

Each of the obtained pharmaceutical preparation was stored at 60°C for a week. To examine the presence or absence of the content reduction of netarsudil after the storage, the residual rate (%) of netarsudil was assessed in the same method as in Test Example 1.

The results are shown in Table 3.

[Table 3]

| | | Reference Example 1 | Reference Example 2 |
|---|---|---|---|
| Aqueous composition (amount: per 100 mL) | Netarsudil dimesylate | 0.02848 g (0.02 g in terms of a free form) | 0.02848 g (0.02 g in terms of a free form) |
| | Sodium hydroxide | q.s. (pH 5.0) | q.s. (pH 5.0) |
| | Purified water | Balance | Balance |
| Presence or absence of immersion of resin piece formed of PET | | Presence | Absence |
| Residual rate (%) | | 88 | 83 |

As shown in the results listed in Table 3, from the comparison between Reference Examples 1 and 2, no content reduction was observed when the piece formed of polyethylene terephthalate was placed in the aqueous composition comprising netarsudil.

From the test results above, it was revealed that when the aqueous composition comprising the compound represented by the formula (1) exemplified by netarsudil or a salt thereof or a solvate thereof is housed in a container formed of a polyester resin exemplified by polyethylene terephthalate, no content reduction due to adsorption or the like under high temperature conditions is observed, and that the container formed of a polyester resin is suitable for housing the aqueous composition.

[Preparation Examples 1 to 36]

Pharmaceutical preparations of Preparation Examples 1 to 36 can be prepared by preparing aqueous compositions each comprising the components in amounts ((g) per 100 mL of the aqueous composition) shown in Tables 4 to 12 with an ordinary method, and housing the prepared aqueous composition each in an eye drop container formed of polyethylene terephthalate.

[Table 4]

| | Preparation Examples | | | |
|---|---|---|---|---|
| | 1 | 2 | 3 | 4 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | | 0.01 | 0.01 | |
| D-mannitol | 4.7 | | | |
| Glycerin | | 2.5 | | |
| Propylene glycol | | | 1.7 | |
| Xylitol | | | 0.7 | |
| Macrogol 6000 | | | | 1 |
| Macrogol 4000 | | | | 4 |
| Sorbitol | | | | |
| Trometamol | | | | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |

(continued)

| | Preparation Examples | | | |
|---|---|---|---|---|
| | 1 | 2 | 3 | 4 |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 5]

| | Preparation Examples | | | |
|---|---|---|---|---|
| | 5 | 6 | 7 | 8 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | | | 1 | 3 |
| Glycerin | | | 3 | |
| Propylene glycol | | | | 1 |
| Xylitol | | | | |
| Macrogol 6000 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |

(continued)

| | Preparation Examples | | | |
|---|---|---|---|---|
| | 5 | 6 | 7 | 8 |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 6]

| | Preparation Examples | | | |
|---|---|---|---|---|
| | 9 | 10 | 11 | 12 |
| Netarsudil dimesylate | 0.01424 g (0.01 g in terms of a free form | 0.05696 g (0.04 g in terms of a free form) | 0.02848 g (0.02 g in terms of a free form) | 0.02848 g (0.02 g in terms of a free form) |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 6000 | 1 | | | |
| Macrogol 4000 | | 2 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Sodium chloride | | 0.1 | | |

(continued)

| | Preparation Examples | | | |
|---|---|---|---|---|
| | 9 | 10 | 11 | 12 |
| Potassium chloride | | | | 0.2 |
| Calcium chloride | | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 7]

| | Preparation Examples | | | |
|---|---|---|---|---|
| | 13 | 14 | 15 | 16 |
| Netarsudil dimesylate | 0.02848 g (0.02 g in terms of a free form) | 0.02848 g (0.02 g in terms of a free form) | 0.01424 g (0.01 g in terms of a free form) | 0.05696 g (0.04 g in terms of a free form) |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 6000 | | 1 | | |
| Macrogol 4000 | | 2 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Sodium chloride | | | 0.05 | |

(continued)

| | Preparation Examples | | | |
|---|---|---|---|---|
| | 13 | 14 | 15 | 16 |
| Potassium chloride | | | 0.05 | |
| Calcium chloride | 0.1 | | | |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.5 | 6.0 | 5.0 |

[Table 8]

| | Preparation Examples | | | |
|---|---|---|---|---|
| | 17 | 18 | 19 | 20 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |
| Acetic acid | | | | |
| Sodium acetate | | | | |
| Sodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | 2.5 | 1 | | 4 |
| Macrogol 4000 | | | | 1 |
| Sorbitol | | | | |
| Trometamol | | | | |
| Benzalkonium chloride | 0.2 | 0.03 | 0.05 | |

(continued)

| | Preparation Examples | | | |
|---|---|---|---|---|
| | 17 | 18 | 19 | 20 |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 5.0 | 5.0 | 5.0 |

[Table 9]

| | Preparation Examples | | | |
|---|---|---|---|---|
| | 21 | 22 | 23 | 24 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | | | 1 | 3 |
| Glycerin | | | 3 | |
| Propylene glycol | | | | 1 |

(continued)

| | Preparation Examples | | | |
|---|---|---|---|---|
| | 21 | 22 | 23 | 24 |
| Xylitol | | | | |
| Macrogol 400 | | | | |
| Macrogol 4000 | | | | |
| Sorbitol | 0.86 | | | |
| Trometamol | | 20 | | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 10]

| | Preparation Examples | | | |
|---|---|---|---|---|
| | 25 | 26 | 27 | 28 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | 0.05 | 0.3 | | |
| Borax | | 0.3 | | |
| Sodium dihydrogen phosphate | | | 0.031 | 0.5 |
| Disodium hydrogen phosphate | | | 0.07 | 0.8 |
| Citric acid | | | | |
| Sodium citrate | | | | |

(continued)

| | Preparation Examples | | | |
|---|---|---|---|---|
| | 25 | 26 | 27 | 28 |
| Acetic acid | | | | |
| Sodium acetate | | | | |
| Sodium edetate | | 0.01 | 0.01 | |
| D-mannitol | 2 | 2 | | 2 |
| Glycerin | 2 | 2 | 1 | |
| Propylene glycol | | | | 1.5 |
| Xylitol | 0.3 | | | |
| Macrogol 400 | | 2 | | |
| Macrogol 4000 | | 1 | | |
| Sorbitol | | | 0.5 | |
| Trometamol | | | 10 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 7.0 | 6.0 | 7.0 | 6.0 |

[Table 11]

| | Preparation Examples | | | |
|---|---|---|---|---|
| | 29 | 30 | 31 | 32 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Latanoprost | 0.005 | | | |
| Travoprost | | 0.004 | | |
| Bimatoprost | | | 0.3 | |
| Tafluprost | | | | 0.0015 |
| Boric acid | | | | |

(continued)

| | Preparation Examples | | | |
|---|---|---|---|---|
| | 29 | 30 | 31 | 32 |
| Borax | | | | |
| Sodium dihydrogen phosphate | | | | |
| Disodium hydrogen phosphate | | | | |
| Citric acid | 0.12 | 0.05 | | |
| Sodium citrate | 0.4 | 0.03 | | |
| Acetic acid | | | 0.001 | |
| Sodium acetate | | | 0.2 | 0.1 |
| Sodium edetate | | 0.01 | 0.01 | |
| D-mannitol | 3 | 1 | 1 | 2 |
| Glycerin | 1 | | 1 | |
| Propylene glycol | | | | 1 |
| Xylitol | | | | |
| Macrogol 400 | | 2 | | |
| Macrogol 4000 | | 1 | | |
| Sorbitol | | | | 0.5 |
| Trometamol | | | 15 | |
| Benzalkonium chloride | 0.015 | 0.03 | 0.05 | |
| Benzododecinium bromide | | | | 0.01 |
| Benzethonium chloride | | | | 0.01 |
| Polyoxyl 40 stearate (Myrj-52) | 0.5 | | | |
| Cremophor RH40 | | 0.5 | | |
| Polyoxyethylene hydrogenated castor oil 40 | | | 0.5 | |
| Polysorbate 80 | | | | 0.5 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 6.0 | 5.5 | 6.0 | 5.5 |

[Table 12]

| | Preparation Examples | | | |
|---|---|---|---|---|
| | 33 | 34 | 35 | 36 |
| Netarsudil dimesylate | 0.02848 g (0.02 g in terms of a free form) | 0.02848 g (0.02 g in terms of a free form) | 0.02848 g (0.02 g in terms of a free form) | 0.02848 g (0.02 g in terms of a free form) |
| Timolol maleate | 6.83 mg (5.0 mg in terms of a free form) | 6.83 mg (5.0 mg in terms of a free form) | 6.83 mg (5.0 mg in terms of a free form) | 6.83 mg (5.0 mg in terms of a free form) |
| Boric acid | 0.04 | 0.05 | | |
| Borax | 0.01 | | | |
| Sodium dihydrogen phosphate | | 0.031 | | |
| Disodium hydrogen phosphate | | 0.07 | | |
| Citric acid | | | 0.05 | |
| Sodium citrate | | | 0.03 | |
| Acetic acid | | | | 0.001 |
| Sodium acetate | | | | 0.2 |
| Sodium edetate | 0.01 | 0.01 | 0.01 | 0.01 |
| D-mannitol | 4.7 | | | |
| Glycerin | | 2.5 | | |
| Propylene glycol | | | 1.7 | |
| Xylitol | | | 0.7 | |
| Macrogol 400 | | 1 | | |
| Macrogol 4000 | | | 1 | |
| Sorbitol | | | | 3 |
| Trometamol | | | | 15 |
| Benzalkonium chloride | 0.015 | 0.03 | 0.05 | |
| Benzododecinium bromide | | | | 0.01 |
| Benzethonium chloride | | | | 0.01 |
| Methyl cellulose | 0.5 | 2 | | |
| Gellan gum | | | 0.2 | 0.6 |
| Sodium hydroxide | q.s. | q.s. | q.s. | q.s. |
| Hydrochloric acid | q.s. | q.s. | q.s. | q.s. |
| Purified water | Total Amount 100 mL | Total Amount 100 mL | Total Amount 100 mL | Total Amount 100 mL |
| pH | 5.0 | 6.0 | 7.0 | 6.0 |

[Preparation Examples 37 to 72]

Pharmaceutical preparations of Preparation Examples 37 to 72 can be prepared in the same way as in Preparation Examples 1 to 36 except for using an eye drop container formed of high-density polyethylene instead of that

formed of polyethylene terephthalate.

[Preparation Examples 73 to 108]

Pharmaceutical preparations of Preparation Examples 73 to 108 can be prepared in the same way as in Preparation Examples 1 to 36 except for using an eye drop container formed of polypropylene instead of that formed of polyethylene terephthalate.

[Preparation Examples 109 to 144]

Pharmaceutical preparations of Preparation Examples 109 to 144 can be prepared in the same way as in Preparation Examples 1 to 36 except for using an eye drop container formed of low-density polyethylene instead of that formed of polyethylene terephthalate.

[Preparation Examples 145 to 288]

Pharmaceutical preparations of Preparation Examples 145 to 288 can be prepared with an ordinary method by using 0.5 g of verosudil monohydrochloride in a free form instead of netarsudil dimesylate in the Preparation Examples 1 to 144.

[Preparation Examples 289 to 432]

Pharmaceutical preparations of Preparation Examples 289 to 432 can be prepared with an ordinary method by using 0.7 g of verosudil monohydrochloride in a free form instead of netarsudil dimesylate in the Preparation Examples 1 to 144.

[Preparation Examples 433 to 576]

Pharmaceutical preparations of Preparation Examples 433 to 576 can be prepared with an ordinary method by using 0.02 g of a compound represented by the formula (8) instead of netarsudil dimesylate in the Preparation Examples 1 to 144.

Industrial Applicability

According to the present invention, a pharmaceutical preparation having excellent stability can be provided, and it can be suitably used in the pharmaceutical industry and the like.

## Claims

1. A pharmaceutical preparation comprising:
an aqueous composition comprising a compound represented by the following formula (1)

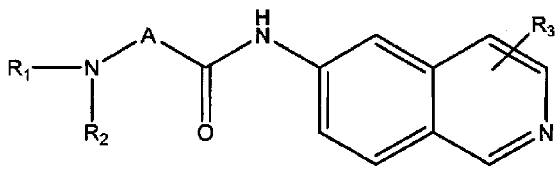

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and
the formula (1) also includes a tautomer thereof) or a salt thereof or a solvate thereof, the aqueous composition being housed in a container formed of a polyester resin or a polyolefin resin.

2. The pharmaceutical preparation according to claim 1, wherein the compound represented by the formula (1) is a

compound represented by the following formula (2), (5), (5') or (8) :

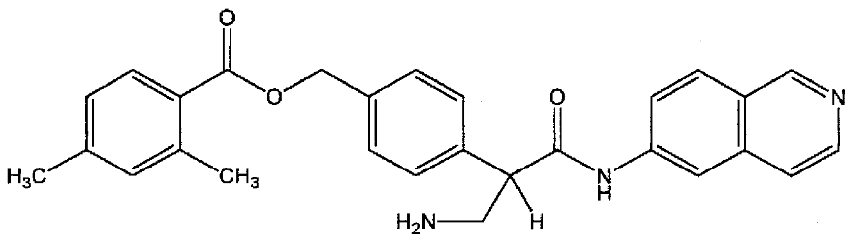

(2) ;

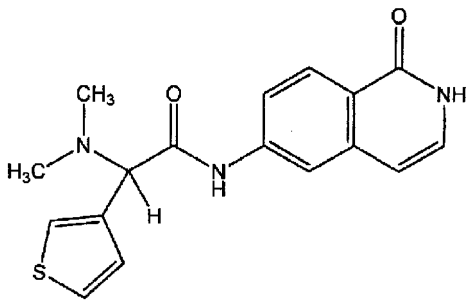

(5) ;

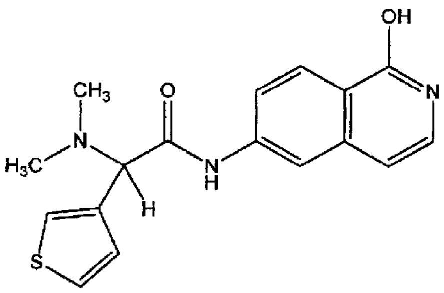

(5') ;

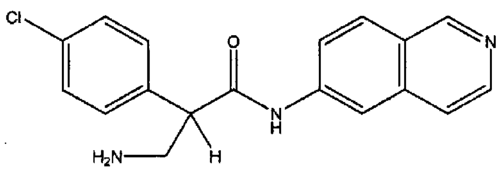

(8) .

3. The pharmaceutical preparation according to claim 1, wherein the compound represented by the formula (1) is a compound represented by the following formula (3):

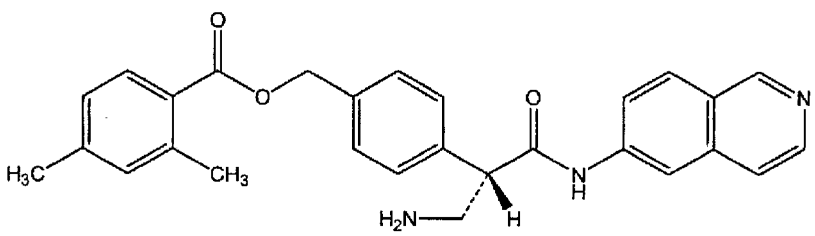

(3) .

4. The pharmaceutical preparation according to any one of claims 1 to 3, wherein the polyester resin is polyethylene terephthalate.

5. The pharmaceutical preparation according to any one of claims 1 to 3, wherein the polyolefin resin is polyethylene

or polypropylene.

**6.** A method of improving heat stability of a compound represented by the following formula (1)

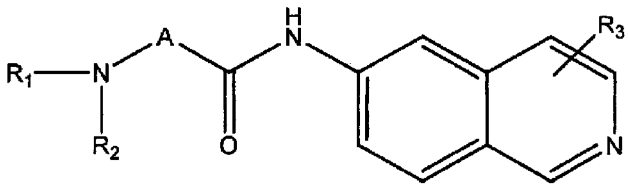

(1)

(in the formula, $R_1$ and $R_2$ each independently represent a hydrogen atom or a $C_1$-$C_4$ alkyl group, $R_3$ represents a hydrogen atom or a hydroxy group, and "A" represents $-CH(R_4)-$ or $-CH_2-CH(R_4)-$ (wherein $R_4$ represents an optionally substituted $C_6$-$C_{10}$ aryl group, or an optionally substituted 5 to 10-membered heteroaryl group), and the formula (1) also includes a tautomer thereof) or a salt thereof or a solvate thereof in an aqueous composition, comprising a step of housing the aqueous composition comprising the compound represented by the formula (1) or a salt thereof or a solvate thereof in a container formed of a polyester resin or a polyolefin resin.

**7.** The method according to claim 6, wherein the compound represented by the formula (1) is a compound represented by the formula (2), (5), (5') or (8):

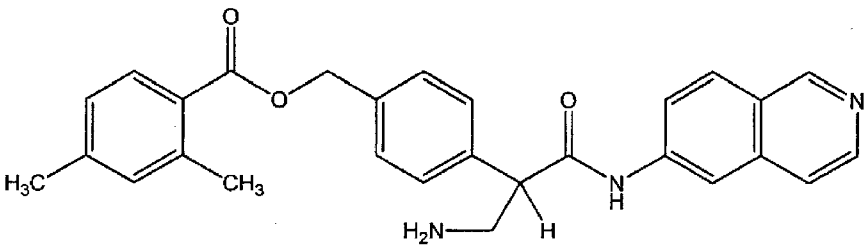

(2) ;

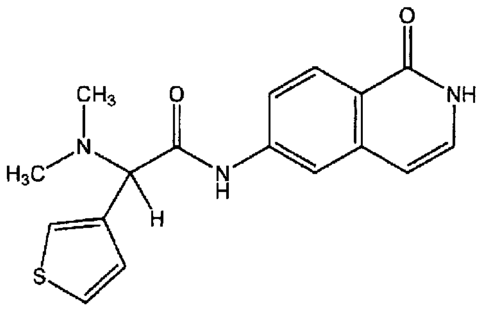

(5) ;

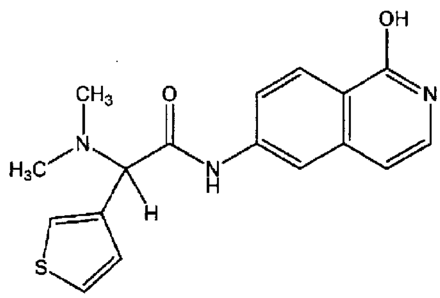

(5') ;

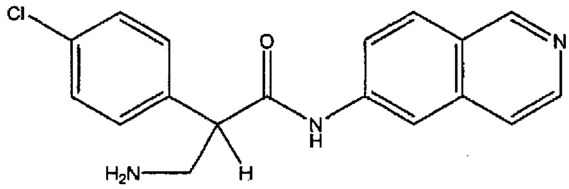

(8) .

8. The method according to claim 6, wherein the compound represented by the formula (1) is a compound represented by the following formula (3):

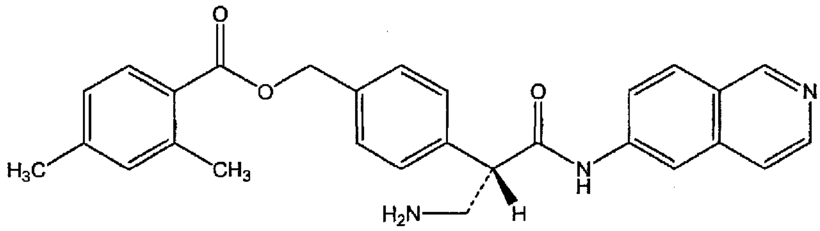

(3) .

9. The method according to any one of claims 6 to 8, wherein the polyester resin is polyethylene terephthalate.

10. The method according to any one of claims 6 to 8, wherein the polyolefin resin is polyethylene or polypropylene.

**INTERNATIONAL SEARCH REPORT**

International application No. PCT/JP2018/007583

A. CLASSIFICATION OF SUBJECT MATTER
Int.Cl. A61K31/472(2006.01)i, A61J1/05(2006.01)i, A61K9/08(2006.01)i, A61K31/4725(2006.01)i, A61K47/04(2006.01)i, A61K47/10(2006.01)i, A61P27/02(2006.01)i, A61P27/06(2006.01)i, B65D1/00(2006.01)i, B65D65/20(2006.01)i

According to International Patent Classification (IPC) or to both national classification and IPC

B. FIELDS SEARCHED

Minimum documentation searched (classification system followed by classification symbols)
Int.Cl. A61K31/472, A61J1/05, A61K9/08, A61K31/4725, A61K47/04, A61K47/10, A61P27/02, A61P27/06, B65D1/00, B65D65/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTY/MEDLINE/EMUASE/BIOSIS (STN), WPI, JSTPlus/JMEDPlus/JST7580 (JDreamIII), Science Direct, PubMed

C. DOCUMENTS CONSIDERED TO BE RELEVANT

| Category* | Citation of document, with indication, where appropriate, of the relevant passages | Relevant to claim No. |
|---|---|---|
| Y | JP 2016-515520 A (AERIE PHARMACEUTICALS, INC.) 30 May 2016, claims, paragraphs [0199], [0200], examples 1-6 & WO 2014/144781 A1, claims, paragraphs [0191], [0192], examples 1-6 & EP 2976080 A1 & US 2014/0275160 A1 | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 March 2018 (23.03.2018) | 03 April 2018 (03.04.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2018/007583

C (Continuation). DOCUMENTS CONSIDERED TO BE RELEVANT

| Category* | Citation of document, with indication, where appropriate, of the relevant passages | Relevant to claim No. |
|---|---|---|
| Y | ELLIS, Eydie Miller et al., "Ocular hypotensive effect of the novel EP3/FP agonist ONO-9054 versus Xalatan: results of a 28-day, double-masked, randomised study", British Journal of Ophthalmology, 20 September 2016, vol. 101, pp. 796-800, Study design, ISSN:1468-2079 | 1-10 |
| A | WO 2016/047720 A1 (KOWA CO., LTD.) 31 March 2016 & US 2017/0266080 A1 & EP 3199162 A1 | 1-10 |
| A | WO 2005/087237 A1 (ASAHI KASEI PHARMA CORPORATION) 22 September 2005 & US 2008/0234483 A1 & EP 1726306 A1 | 1-10 |
| A | JP 2016-138085 A (ROHTO PHARMACEUTICAL CO., LTD.) 04 August 2016 (Family: none) | 1-10 |
| A | LEVY, Brian et al., "Ocular hypotensi ve safety and systemic absorption of AR-13324 ophthalmic solution in normal volunteers", American Journal of Ophthalmology, May 2015, vol. 159, no. 5, pp. 980-985.el, ISSN:0002-9394 | 1-10 |
| A | REN, Ruiyi et al., "Netarsudil increases outflow facility in human eyes through multiple mechanisms", Investigative Ophthalmology and Visual Science, November 2016, vol. 57, no. 14, pp. 6197-6209, ISSN:1552-5783 | 1-10 |
| P, X | RHOPRESSA™ (netarsudil ophthalmic solution) Label, NDA 208254, Reference ID:4194833, December 2017, pp. 4-10 | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2012525386 A
- WO 2009091898 A
- JP 2016515520 A

### Non-patent literature cited in the description

- **BACHARACH J. et al.** *Ophthalmology,* 2015, vol. 122 (2), 302-7
- **STURDIVANT JM. et al.** *Bioorg Med Chem Lett.,* 2016, vol. 26 (10), 2475-80
- **WILLIAMS RD. et al.** *Am. J. Ophthalmol.,* 2011, vol. 152 (5), 834-41
- General Notices. Japanese Pharmacopoeia
- General Rules for Preparation. Japanese Pharmacopoeia